# EUROPEAN PATENT SPECIFICATION

(11) **EP 2 156 184 B1**
(45) Date of publication and mention of the grant of the patent: **27.11.2013**
(21) Application number: 08754314.6
(22) Date of filing: 08.05.2008
(51) Int. Cl.: G01N 33/53, G01N 33/574

(54) **METHODS FOR DIAGNOSING AND TREATING PROSTATE AND LUNG CANCER**
VERFAHREN ZUR DIAGNOSTIZIERUNG UND BEHANDLUNG VON PROSTATA- UND LUNGENKREBS
PROCEDES DE DIAGNOSTIC ET DE TRAITEMENT DU CANCER DE LA PROSTATE ET DU CANCER DU POUMON

(30) Priority: 08.05.2007 US 916719 P
(43) Date of publication of application: 24.02.2010
(73) Proprietor: Picobella, LLC, Burlingame, CA 94010 (US)
(72) Inventor: WANG, Bruce, Mountain View, CA 94040 (US); WABL, Matthias, San Francisco, CA 94122 (US)
(74) Representative: Murray, Adrian D'Coligny
(86) International application number: PCT/US2008/005983
(87) International publication number: WO 2008/140774

(56) References cited:
- WO-A2-02/074960
- WO-A2-02/086443
- WO-A2-03/009814
- WO-A2-2007/047796
- US-A1- 2003 108 963
- US-A1- 2006 094 046
- US-A1- 2006 211 059
- US-A1- 2007 010 726

### SEQUENCE LISTING

<110> PICOBELLA LLC wang, Bruce wabl, Matthias
<120> METHODS FOR DIAGNOSING AND TREATING
   PROSTATE AND LUNG CANCER
<130> 598498015wo0
<140> To be Assigned
   <141> Herewith
<150> US 60/916,719
   <151> 2007-05-08
<160> 4
<170> FastSEQ for Windows Version 4.0
<210> 1
   <211> 590
   <212> PRT
   <213> Homo sapiens
<400> 1
   Met Lys Val Gly Val Leu Trp Leu Ile Ser Phe Phe Thr Phe Thr Asp
   1               5                   10                  15
   Gly His Gly Gly Phe Leu Gly Lys Asn Asp Gly Ile Lys Thr Lys Lys
               20                  25                  30
   Glu Leu Ile Val Asn Lys Lys Lys His Leu Gly Pro Val Glu Glu Tyr
           35                  40                  45
   Gln Leu Leu Leu Gln Val Thr Tyr Arg Asp Ser Lys Glu Lys Arg Asp
       50                  55                  60
   Leu Arg Asn Phe Leu Lys Leu Leu Lys Pro Pro Leu Leu Trp Ser His
   65                  70                  75                  80
   Gly Leu Ile Arg Ile Ile Arg Ala Lys Ala Thr Thr Asp Cys Asn Ser
                   85                  90                  95
   Leu Asn Gly Val Leu Gln Cys Thr Cys Glu Asp Ser Tyr Thr Trp Phe
               100                 105                 110
   Pro Pro Ser Cys Leu Asp Pro Gln Asn Cys Tyr Leu His Thr Ala Gly
           115                 120                 125
   Ala Leu Pro Ser Cys Glu Cys His Leu Asn Asn Leu Ser Gln Ser Val
       130                 135                 140
   Asn Phe Cys Glu Arg Thr Lys Ile Trp Gly Thr Phe Lys Ile Asn Glu
   145                 150                 155                 160
   Arg Phe Thr Asn Asp Leu Leu Asn Ser Ser Ser Ala Ile Tyr Ser Lys
                   165                 170                 175
   Tyr Ala Asn Gly Ile Glu Ile Gln Leu Lys Lys Ala Tyr Glu Arg Ile
               180                 185                 190
   Gln Gly Phe Glu Ser Val Gln Val Thr Gln Phe Arg Asn Gly Ser Ile
           195                 200                 205
   Val Ala Gly Tyr Glu Val Val Gly Ser Ser Ser Ala Ser Glu Leu Leu
       210                 215                 220
   Ser Ala Ile Glu His Val Ala Glu Lys Ala Lys Thr Ala Leu His Lys
   225                 230                 235                 240
   Leu Phe Pro Leu Glu Asp Gly Ser Phe Arg Val Phe Gly Lys Ala Gln
                   245                 250                 255
   Cys Asn Asp Ile Val Phe Gly Phe Gly Ser Lys Asp Asp Glu Tyr Thr
               260                 265                 270
   Leu Pro Cys Ser Ser Gly Tyr Arg Gly Asn Ile Thr Ala Lys Cys Glu
           275                 280                 285
   Ser Ser Gly Trp Gln Val Ile Arg Glu Thr Cys Val Leu Ser Leu Leu
       290                 295                 300
   Glu Glu Leu Asn Lys Asn Phe Ser Met Ile Val Gly Asn Ala Thr Glu
   305                 310                 315                 320
   Ala Ala Val Ser Ser Phe Val Gln Asn Leu Ser Val Ile Ile Arg Gln
                   325                 330                 335
   Asn Pro Ser Thr Thr Val Gly Asn Leu Ala Ser Val Val Ser Ile Leu
               340                 345                 350
   Ser Asn Ile Ser Ser Leu Ser Leu Ala Ser His Phe Arg Val Ser Asn
           355                 360                 365
   Ser Thr Met Glu Asp Val Ile Ser Ile Ala Asp Asn Ile Leu Asn Ser
       370                 375                 380
   Ala Ser Val Thr Asn Trp Thr Val Leu Leu Arg Glu Glu Lys Tyr Ala
   385                 390                 395                 400
   Ser Ser Arg Leu Leu Glu Thr Leu Glu Asn Ile Ser Thr Leu Val Pro
                   405                 410                 415
   Pro Thr Ala Leu Pro Leu Asn Phe Ser Arg Lys Phe Ile Asp Trp Lys
               420                 425                 430
   Gly Ile Pro Val Asn Lys Ser Gln Leu Lys Arg Gly Tyr Ser Tyr Gln
           435                 440                 445
   Ile Lys Met Cys Pro Gln Asn Thr Ser Ile Pro Ile Arg Gly Arg Val
       450                 455                 460
   Leu Ile Gly Ser Asp Gln Phe Gln Arg Ser Leu Pro Glu Thr Ile Ile
   465                 470                 475                 480
   Ser Met Ala Ser Leu Thr Leu Gly Asn Ile Leu Pro Val Ser Lys Asn
                   485                 490                 495
   Gly Asn Ala Gln Val Asn Gly Pro Val Ile Ser Thr Val Ile Gln Asn
               500                 505                 510
   Tyr Ser Ile Asn Glu Val Phe Leu Phe Phe Ser Lys Ile Glu Ser Asn
           515                 520                 525
   Leu Ser Gln Pro His Cys Val Phe Trp Asp Phe Ser His Leu Gln Trp
       530                 535                 540
   Asn Asp Ala Gly Cys His Leu Val Asn Glu Thr Gln Asp Ile Val Thr
   545                 550                 555                 560
   Cys Gln Cys Thr His Leu Thr Ser Phe Ser Ile Leu Met Ser Pro Phe
                   565                 570                 575
   Val Pro Ser Thr Ile Phe Pro Val Val Lys Trp Ile Thr Tyr
               580                 585                 590
<210> 2
   <211> 910
   <212> PRT
   <213> Homo sapiens
<400> 2
   Met Lys Val Gly Val Leu Trp Leu Ile Ser Phe Phe Thr Phe Thr Asp
   1               5                   10                  15
   Gly His Gly Gly Phe Leu Gly Lys Asn Asp Gly Ile Lys Thr Lys Lys
               20                  25                  30
   Glu Leu Ile Val Asn Lys Lys Lys His Leu Gly Pro Val Glu Glu Tyr
           35                  40                  45
   Gln Leu Leu Leu Gln Val Thr Tyr Arg Asp Ser Lys Glu Lys Arg Asp
       50                  55                  60
   Leu Arg Asn Phe Leu Lys Leu Leu Lys Pro Pro Leu Leu Trp Ser His
   65                  70                  75                  80
   Gly Leu Ile Arg Ile Ile Arg Ala Lys Ala Thr Thr Asp Cys Asn Ser
                   85                  90                  95
   Leu Asn Gly Val Leu Gln Cys Thr Cys Glu Asp Ser Tyr Thr Trp Phe
               100                 105                 110
   Pro Pro Ser Cys Leu Asp Pro Gln Asn Cys Tyr Leu His Thr Ala Gly
           115                 120                 125
   Ala Leu Pro Ser Cys Glu Cys His Leu Asn Asn Leu Ser Gln Ser Val
       130                 135                 140
   Asn Phe Cys Glu Arg Thr Lys Ile Trp Gly Thr Phe Lys Ile Asn Glu
   145                 150                 155                 160
   Arg Phe Thr Asn Asp Leu Leu Asn Ser Ser Ser Ala Ile Tyr Ser Lys
                   165                 170                 175
   Tyr Ala Asn Gly Ile Glu Ile Gln Leu Lys Lys Ala Tyr Glu Arg Ile
               180                 185                 190
   Gln Gly Phe Glu Ser Val Gln Val Thr Gln Phe Arg Asn Gly Ser Ile
           195                 200                 205
   Val Ala Gly Tyr Glu Val Val Gly Ser Ser Ser Ala Ser Glu Leu Leu
       210                 215                 220
   Ser Ala Ile Glu His Val Ala Glu Lys Ala Lys Thr Ala Leu His Lys
   225                 230                 235                 240
   Leu Phe Pro Leu Glu Asp Gly Ser Phe Arg Val Phe Gly Lys Ala Gln
                   245                 250                 255
   Cys Asn Asp Ile Val Phe Gly Phe Gly Ser Lys Asp Asp Glu Tyr Thr
               260                 265                 270
   Leu Pro Cys Ser Ser Gly Tyr Arg Gly Asn Ile Thr Ala Lys Cys Glu
           275                 280                 285
   Ser Ser Gly Trp Gln Val Ile Arg Glu Thr Cys Val Leu Ser Leu Leu
       290                 295                 300
   Glu Glu Leu Asn Lys Asn Phe Ser Met Ile Val Gly Asn Ala Thr Glu
   305                 310                 315                 320
   Ala Ala Val Ser Ser Phe Val Gln Asn Leu Ser Val Ile Ile Arg Gln
                   325                 330                 335
   Asn Pro Ser Thr Thr Val Gly Asn Leu Ala Ser Val Val Ser Ile Leu
               340                 345                 350
   Ser Asn Ile Ser Ser Leu Ser Leu Ala Ser His Phe Arg Val Ser Asn
           355                 360                 365
   Ser Thr Met Glu Asp Val Ile Ser Ile Ala Asp Asn Ile Leu Asn Ser
       370                 375                 380
   Ala Ser Val Thr Asn Trp Thr Val Leu Leu Arg Glu Glu Lys Tyr Ala
   385                 390                 395                 400
   Ser Ser Arg Leu Leu Glu Thr Leu Glu Asn Ile Ser Thr Leu Val Pro
                   405                 410                 415
   Pro Thr Ala Leu Pro Leu Asn Phe Ser Arg Lys Phe Ile Asp Trp Lys
               420                 425                 430
   Gly Ile Pro Val Asn Lys Ser Gln Leu Lys Arg Gly Tyr Ser Tyr Gln
           435                 440                 445
   Ile Lys Met Cys Pro Gln Asn Thr Ser Ile Pro Ile Arg Gly Arg Val
       450                 455                 460
   Leu Ile Gly Ser Asp Gln Phe Gln Arg Ser Leu Pro Glu Thr Ile Ile
   465                 470                 475                 480
   Ser Met Ala Ser Leu Thr Leu Gly Asn Ile Leu Pro Val Ser Lys Asn
                   485                 490                 495
   Gly Asn Ala Gln Val Asn Gly Pro Val Ile Ser Thr Val Ile Gln Asn
               500                 505                 510
   Tyr Ser Ile Asn Glu Val Phe Leu Phe Phe Ser Lys Ile Glu Ser Asn
           515                 520                 525
   Leu Ser Gln Pro His Cys Val Phe Trp Asp Phe Ser His Leu Gln Trp
       530                 535                 540
   Asn Asp Ala Gly Cys His Leu Val Asn Glu Thr Gln Asp Ile Val Thr
   545                 550                 555                 560
   Cys Gln Cys Thr His Leu Thr Ser Phe Ser Ile Leu Met Ser Pro Phe
                   565                 570                 575
   Val Pro Ser Thr Ile Phe Pro Val Val Lys Trp Ile Thr Tyr Val Gly
               580                 585                 590
   Leu Gly Ile Ser Ile Gly Ser Leu Ile Leu Cys Leu Ile Ile Glu Ala
           595                 600                 605
   Leu Phe Trp Lys Gln Ile Lys Lys Ser Gln Thr Ser His Thr Arg Arg
       610                 615                 620
   Ile Cys Met Val Asn Ile Ala Leu Ser Leu Leu Ile Ala Asp Val Trp
   625                 630                 635                 640
   Phe Ile Val Gly Ala Thr Val Asp Thr Thr Val Asn Pro Ser Gly Val
                   645                 650                 655
   Cys Thr Ala Ala Val Phe Phe Thr His Phe Phe Tyr Leu Ser Leu Phe
               660                 665                 670
   Phe Trp Met Leu Met Leu Gly Ile Leu Leu Ala Tyr Arg Ile Ile Leu
           675                 680                 685
   Val Phe His His Met Ala Gln His Leu Met Met Ala Val Gly Phe Cys
       690                 695                 700
   Leu Gly Tyr Gly Cys Pro Leu Ile Ile Ser Val Ile Thr Ile Ala Val
   705                 710                 715                 720
   Thr Gln Pro Ser Asn Thr Tyr Lys Arg Lys Asp Val Cys Trp Leu Asn
                   725                 730                 735
   Trp Ser Asn Gly Ser Lys Pro Leu Leu Ala Phe Val Val Pro Ala Leu
               740                 745                 750
   Ala Ile Val Ala Val Asn Phe Val Val Val Leu Leu Val Leu Thr Lys
                   755                     760                     765
   Leu Trp Arg Pro Thr Val Gly Glu Arg Leu Ser Arg Asp Asp Lys Ala
       770                     775                     780
   Thr Ile Ile Arg Val Gly Lys Ser Leu Leu Ile Leu Thr Pro Leu Leu
   785                     790                     795                 800
   Gly Leu Thr Trp Gly Phe Gly Ile Gly Thr Ile Val Asp Ser Gln Asn
                       805                     810                 815
   Leu Ala Trp His Val Ile Phe Ala Leu Leu Asn Ala Phe Gln Gly Phe
                   820                     825                     830
   Phe Ile Leu Cys Phe Gly Ile Leu Leu Asp Ser Lys Leu Arg Gln Leu
               835                     840                     845
   Leu Phe Asn Lys Leu Ser Ala Leu Ser Ser Trp Lys Gln Thr Glu Lys
       850                     855                     860
   Gln Asn Ser Ser Asp Leu Ser Ala Lys Pro Lys Phe Ser Lys Pro Phe
   865                     870                     875                 880
   Asn Pro Leu Gln Asn Lys Gly His Tyr Ala Phe Ser His Thr Gly Asp
                       885                     890                     895
   Ser Ser Asp Asn Ile Met Leu Thr Gln Phe Val Ser Asn Glu
               900                     905                     910
<210> 3
   <211> 19
   <212> PRT
   <213> Homo sapiens
<400> 3
   Asp Leu Phe Ile Asp Lys Lys Val Leu Lys Val Ala His Val Glu His
    1               5                  10                  15
   Glu Glu Thr
<210> 4
   <211> 16
   <212> PRT
   <213> Homo sapiens
<400> 4
   Leu Ala Tyr Asp Leu Asp Val Asp Asp Ala Pro Gly Asn Ser Gln Gln
    1               5                  10                  15

## Description

### Field of the Invention

The present invention relates to a method of screening for prostate and lung cancer in a human subject.

### References

The following references are cited below In support of the background of the invention or methods employed in practicing the invention.
1. Bjarnadottir TK, Geirardsdottir K, Ingemansson M, Mirza MA, Fredriksson R, Schioth HB. Identification of novel splice variants of Adhesion G protein-coupled receptors. Gene. 2007 Jan 31;387(1-2):38-48. Epub 2006 Aug 30.
2. Bjarnadottir TK, Fredriksson R, Hoglund PJ, Gloriam DE, Lagerstrom MC, Schioth HB. The human and mouse repertoire of the adhesion family of G-protein-coupled receptors. Genomics. 2004 Jul;84(1):23-33.
3. Fredriksson R, Lagerstrom MC, Hoglund PJ, Schioth HB. Novel human G protein-coupled receptors with long N-terminals containing GPS domains and Ser/Thr-rich regions. FEBS Lett. 2002 Nov 20;531 (3):407-14.
4. Nusse, R., van Ooyen, A., Cox, D., Fung, Y. K. & Varmus, H. Mode of proviral activation of a putative mammary oncogene (int-1) on mouse chromosome 15. Nature 307, 131-6 (1984).
5. Nusse, R. & Varmus, H. E. Many tumors Induced by the mouse mammary tumor virus contain a provirus integrated in the same region of the host genome. Cell 31, 99-109 (1982).
6. Sorensen, A. B., Duch, M., Amtoft, H. W., Jorgensen, P. & Pedersen, F. S. Sequence tags of provirus integration sites in DNAs of tumors induced by the murine retrovirus SL3-3. J Virol 70, 4063-70 (1996).
7. Lund, A. H. et al. Genome-wide retroviral Insertional tagging of genes involved in cancer in Cdkn2a-deficient mice. Nat Genet 32, 160-5 (2002).
8. Mikkers, H. et al. High-throughput retroviral tagging to identify components of specific signaling pathways in cancer. Nat Genet 32, 153-9 (2002).
9. Collier, L. S., Carlson, C. M., Ravimohan, S., Dupuy, A. J. & Largaespada, D. A. Cancer gene discovery in solid tumours using transposon-based somatic mutagenesis in the mouse. Nature 436, 272-6 (2005).
10. Dupuy, A. J., Akagi, K., Largaespada, D. A., Copeland, N. G. & Jenkins, N. A. Mammalian mutagenesis using a highly mobile somatic Sleeping Beauty transposon system. Nature 436, 221-6 (2005).
11. Wang, et al., Nucleic Acids Research, (England) 2005, Vol. 33, p.21.
12. Oh da Y, Kim K, Kwon HB, Seong JY. Cellular and molecular biology of orphan G protein-coupled receptors.Int Rev Cytol. 2006;252:163-218.
13. Lundstrom K. Latest development in drug discovery on G protein-coupled receptors.Curr Protein Pept Sci. 2006 Oct;7(5):465-70.
14. Jacoby E, Bouhelal R, Gerspacher M, Seuwen K. The 7 TM G-protein-coupled receptor target family. ChemMedChem. 2006 Aug;1(8):761-82.

### Background of the Invention

Prostate cancer is the most common malignant cancer in North American men. It is estimated that approximately 200,000 new cases and 31,500 prostate cancer-related deaths will occur in the United States annually. Prostate cancer is now the second leading cause of cancer death in men, exceeded only by lung cancer. It accounts for 29% of all male cancers and 11 % of male cancer-related deaths.

Currently, the FDA has approved serum PSA (prostate-specific antigen) for use as a prostate cancer screening laboratory test. Like many serum tumor markers, PSA is produced by both normal and cancerous glands. In men with prostate cancer, the serum levels can be elevated with both localized and advanced or disseminated disease. PSA levels are generally proportional to the volume of the cancer. Because there is a significant overlap between PSA levels found in cancer and benign prostatic hyperplasia, it is important to obtain sequential levels in low or borderline elevated values.

The introduction of free PSA (fPSA) testing has introduced a greater level of specificity in identifying early prostate cancer. In 1998, the FDA approved fPSA testing as a diagnostic aid for men with total PSA values between 4.0-10.0 ng/mL. This has often been the diagnostic gray zone for total PSA testing and fPSA may aid in the stratification. In general, at any free PSA level, the more enlarged the prostate, the more likely the prostate may be cancerous. However, these tests remain qualitative at best, and more reliable types of detection, and means for staging the cancer treatment, are needed.

Prostate cancer, like other forms of cancer, is caused by genetic aberrations, i.e., mutations. In mutant cells the normal balance between the factors that promote and restrain growth is disrupted, and as a result, these mutant cells proliferate continuously-the hallmark of tumor cells. Mutations can arise spontaneously or by external factors such as chemical mutagens, radiation, or viral integration, which inserts extra-genomic DNA that may or may not contain an oncogene. A cellular gene can be modified by point mutation, insertion and frame shift (including truncation), (functional) deletion (including silencing), or translocation, which sometimes can result in gene fusion. In this way protooncogenes can become oncogenes, which promote proliferation, and tumor suppressor genes can become inactivated, also inducing tumor growth. Any combination of the above-mentioned changes in DNA can contribute to tumor formation. The consequences of these changes may or may not be held in check by the immune system (immune surveillance).

Various methods of diagnosis are known in the prior art. For example, WO02/086443 discloses methods and compositions that can be used for diagnosis and treatment of lung cancer and similar pathologies. The document provides various nucleotide sequences of genes that are up- and down-regulated in lung cancer cells, which may be useful for diagnostic purposes and as targets for screening therapeutic compounds that modulate lung cancer. WO02/074960 also provides isolated nucleic acid molecules which encode various proteins. Diagnostic methods are also provided.

WO03/009814 discloses nucleic acid molecules and proteins associated with prostate cancer including pre-malignant conditions. Compositions, kits and methods for detecting, characterizing, preventing and treating human prostate cancers are also provided. WO2007/047796 discloses tissue-derived glycoproteins and glycosites detectable in plasma and methods for detecting disease using such molecules.

Heretofore, there has been no demonstrated link between changes in blood or serum GPR110 levels and prostate or lung cancer. Such a link could have a number of important diagnostic and therapeutic applications. In accordance with the present invention, it has now been discovered that (i) GPR110 levels increase significantly in prostate and lung cancer cells, and (ii) this increase can be measured in blood or urine-fluid sample of patients.

### Summary of the Invention

The invention provides a method for screening for lung or prostate cancer in a human subject comprising assaying the level of human GPR110 in a subject blood or serum sample, includes contacting said sample with an anti-GPR110 antibody specific against a GPR110 epitope represented by amino acid residues within SEQ ID NO:1, under conditions effective to bind the antibody to the GPR110 epitope, separating antibody bound to the GPR110 epitope from unbound antibody, and detecting the level of the antibody bound to the GPR110 epitope; determining if the detected level of antibody bound to the GPR110 epitope is at least threefold greater than that of anti-GPR110 antibody bound to the GPR110 epitope present in blood or serum samples obtained from normal individuals.

Optionally, the method may include screening for the presence of lung or prostate cancer by means of an independent test for lung or prostate cancer, respectively, in the subject, if the assayed level is at least threefold greater than normal level, where the independent test may be carried out prior to, contemporaneous with, or following steps (a) and (b).

Step (a) may include applying the blood or serum sample body fluid to a solid-phase immunoassay device, where the level of GPR110 in the sample is indicated qualitatively by a colorimetric or fluorometric indicator, and the determining step includes comparing the indicator with a known standard.

In another aspect, the invention includes an improvement in a method for detecting the presence of lung or prostate cancer, by detection of a depressed or elevated level of a biological marker that is diagnostic of the lung or prostate cancer.

For example, the improvement may be used in a method for detecting prostate cancer in a human male subject, by reacting a subject body-fluid sample with an antibody specific against at least one marker protein selected from one of total prostate specific antigen (PSA), free PSA, and glypican 3 protein (GPC3), and determining, as an indicator of prostate cancer, whether the subject has an increased level of at least one of said marker protein.

In still another aspect, the invention contemplates the use of a measured value of GPR110 and a measured value of at least one marker antigen selected from total prostate specific antigen (PSA), free PSA, and glypican 3 protein (GPC3) in a blood or serum sample from a human subject for screening the subject for the presence of prostate cancer.

The GPR110 antibody may be a human or humanized anti-GPR110 antibody specific against an epitope contained within SEQ ID NO:1. The antibody may be effective, when bound to GPR110 on the surface of prostate or lung cancer cells, to promote antibody-dependent cell cytotoxicity. The antibody may have conjugated thereto, a therapeutic agent effective to kill or inhibit cancer cells, when the agent becomes bound to or incorporated into said cells.

These and other aspects, objects, advantages, and features of the invention will become apparent to those persons skilled in the art upon reading the details of the invention as more fully described below.

### Brief Descriptions of the Drawings

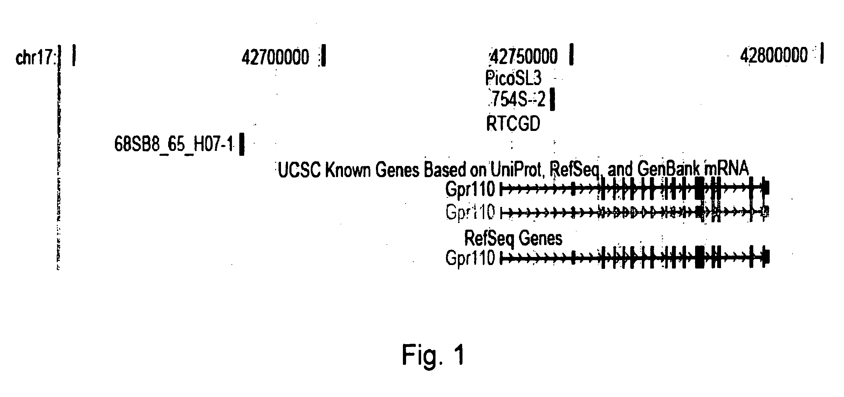

Fig. 1 shows the genomic organization of the mouse Gpr110 locus, as view by a customized screen print of the UCSC genome web site browser (February 2006 version of the mm8 gene assembly). Top, base position on chromosome 17. The green vertical handle bar below "PicoSL3" represents a retroviral integration into the locus identified from a single tumor (754S-2). A public domain integration site (68SB8_65_H07-1) is indicated below "RTCGD".

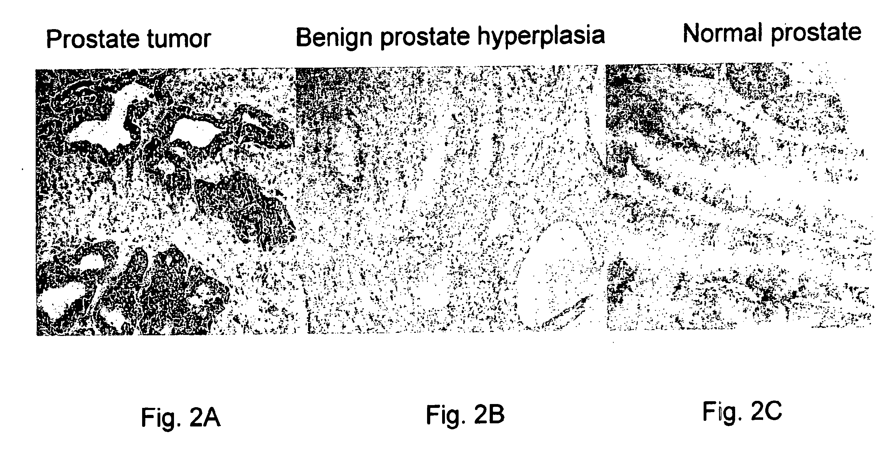

Figs. 2A-2C show immunohistochemical stains (brown) of human prostate tumor (2A), benign prostate hyperplasia (2B), and normal tissue (2C). No or low expression is generally seen in normal tissue or benign prostate hyperplasia, while significant overexpression is seen in tumor tissue. The polyclonal rabbit antibody serum reacts with an epitope found within amino acid residues 1-590 of human GPR110, defined herein as SEQ ID NO: 1.

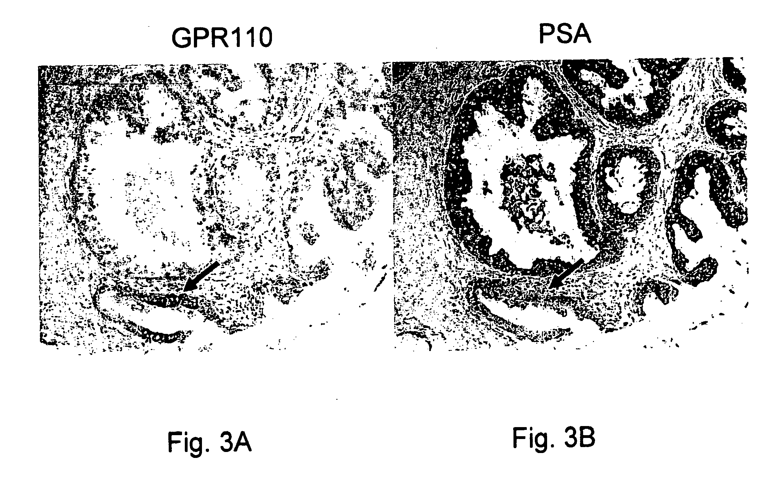

Figs. 3A and 3B show immunohistochemical stains (brown) of human benign prostate hyperplasia tissue stained with anti-GPR110 antibodies (3A) and anti-PSA antibodies (3B). The arrow points to a small group of cancer stem cells that are GPR110-positive and PSA-negative. The GPR110 peptide serum used is the same as described in Fig. 2.

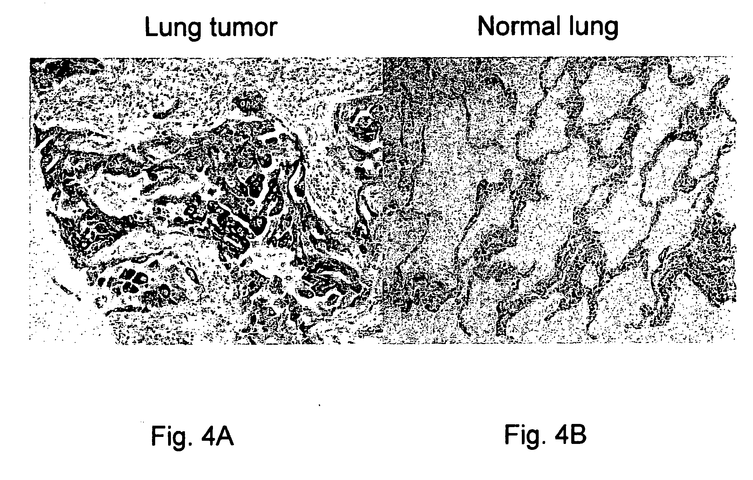

Figs. 4A and 4B show immunohistochemical stains (brown) of human lung tumor (4A) and normal tissue (4B). No or low expression is seen in normal tissue while significant overexpression is seen in tumor tissue. The peptide serum used is the same as described in Fig. 2.

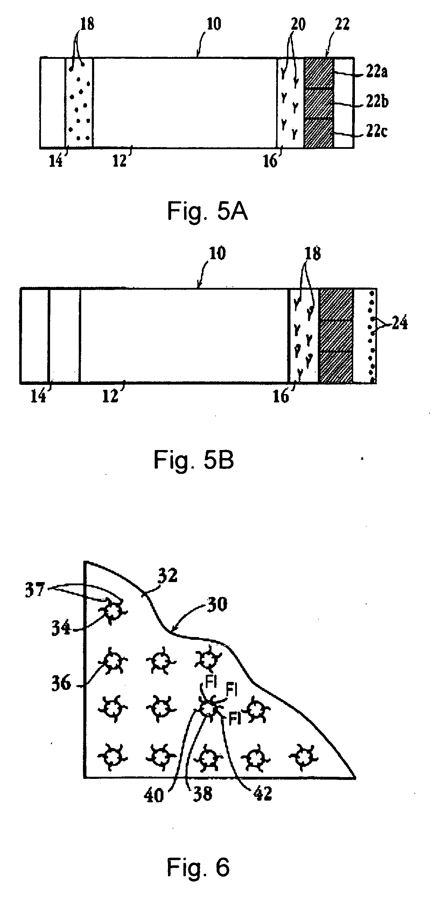

Figs. 5A and 5B show a solid-phase diagnostic device for determining GPR110 levels in a human patient, at initial (5A) and final stages (5B) of the assay.

### Detailed Description of the Invention

### A. Definitions

The following terms have the definitions given below, unless otherwise indicated in the specification.

"Screening" for cancer means diagnostic information that either alone, or in combination with other diagnostic information, can be used to determine the presence or absence of a cancer, or the increased likelihood of a cancer, or used to classify the type of cancer, e.g., a lung cancer characterized by elevated levels of GPR110 expression.

"Other indicator that is diagnostic of lung or prostate cancer" refers to a diagnostic test, other than a biological marker that can be used to detect or characterize the presence or extent or type of a cancer. Exemplary indicators include imaging data obtained by X-ray, CT scan, or MRI imaging methods, or histological observations on blopsied tissue.

"Staging" treatment of cancer, in accordance with the present invention, involves determining the stage of cancer in an individual, based on the level of GPR110 detected, and tailoring the treatment to that stage. There are four recognized stages of cancer, which are defined by the degree of localization and organization of cancer cells. In addition, cancer may be defined as early stage at which the cancer is responsive to a number of hormonal-based therapies, and a later, more serious androgen-independent stage.

An "assayed level of GPR110" refers to an assayed level of wildtype human GPR110, or a variant, e.g., splice variant or mutated form of the protein, or a GPR110 fragment.

An "assayed level of human GPR110 transcript" refers to an assayed level of RNA transcript encoding wildtype human GPR110, or a variant, e.g., splice variant or mutated form of the protein, or a GPR110 fragment.

An "increased" or "above-normal" level of GPR110 refers to a level of the protein, or fragment or variant thereof, as determined, for example, by immunochemical staining or detection that is at least about 50 percent higher than the value of the detectable level of the protein measured in a population of normal (non-cancerous) individuals. Preferably, the level of GPR110 is at least three times higher than the GPR110 value for a similar sample from a normal patient.

An "increased" or "above-normal" level of GPR110 RNA transcript refers to a level amount of the transcript, as determined, for example, by PCR amplification and transcript separation that is at least about 50 percent higher than the value of the detectable level of the transcript measured in a population of normal (non-cancerous) individuals. Preferably, the level of GPR110 is at least three times higher than the GPR110 value for a similar sample from a normal patient.

"The value of the detectable level of GPR110 protein or its RNA transcript measured in a population of normal (non-cancerous) individuals" may refer, for example, to the statistical mean or average of such values for a population, e.g., 5 or more, preferably 10 or more normal individuals, or may refer to the highest value recorded for the GPR110 protein or transcript for the individuals in the population of normal individuals. Such values are readily determined by assaying GPR110 or its transcript from a selected sample source, e.g., lung or prostate tissue, or a blood or serum sample, using assay methods described below. It will be understood that normal values are determined from the same type of tissue, e.g., lung or prostate tissue, or sample source, e.g., a blood or serum sample, as the tissue or sample source being assayed for the presence of elevated levels of GPR110 or its transcript.

"GPR110 assay" refers to an assay that measures the level or presence of GPR110 protein, either in wildtype or variant form, or an epitope thereof, or measures the level of an RNA transcript that encodes GPR110 protein or a fragment thereof.

### B. GPR110 Protein and Expression

The human GPR110 gene encodes a putative orphan "adhesion class" G protein-coupled receptor whose biological function and natural ligand(s) are unknown (refs. 1-3). The human GPR110 gene has two known isoforms and is found at chromosome region 6p12.3. Isoform 1 (NM_153840.2) encodes a putative protein (NP_722582.2) having 910 amino acids (AA) and a calculated molecular weight (MW) of 101234 Da. Isoform 2 (NM_025048.2) encodes a putative protein (NP_079324.2) having 218 AA, a calculated MW of 24745 Da, and a unique C-terminus compared to isoform 1. The mouse Gpr110 gene (NM_133776.1) is found at chromosome region 17 B3; the encoded protein (NP_598537.1) has 908 AA and a calculated MW of 101338 Da. The human GPR110 protein is a putative cell surface 7 transmembrane protein, and contains a G-protein-coupled receptor proteolytic site (GPS) domain and an SEA domain, as well as several possible N-linked glycosylation sites near the N-terminus.

### C. Identification of GPR110 as a Cancer Gene

Cancer genes (oncogenes and tumor suppressor genes) were defined in a high throughput manner by using proviral tagging. Although viruses have not yet been implicated as a major cause of cancers in humans, research using tumor viruses has led to the discovery of many oncogenes and protooncogenes. In proviral tagging, mice are infected with a retrovirus that does not contain an oncogene (*e.g*., murine leukemia virus, MLV or murine mammary tumor virus, MMTV (4-8). Recently, the host range of this approach has been broadened by the use of a transposon (9, 10).

During retroviral infection, the virus integrates into the cellular genome and inserts its DNA near or within genes, which leads to various outcomes: (i) the insertion site is too far away from a protooncogene and thus does not activate it. In this case, there will be no selection for that cell. (ii) The provirus inserts within 200 kb of a protooncogene, but not within the gene (type 1). Here, either the viral promoter or the viral enhancer increases the expression level of the protooncogene. (iii) The provirus inserts within a gene, destroying or altering its function (type 2). There will be no selection for a cell that contains either type 1 or type 2 insertion events in a gene that is not a protooncogene or tumor suppressor gene. If integration results in the formation of a tumor, genes adjacent to the integration site can be identified, and classified as either protooncogenes or tumor suppressor genes. This method has been used to identify many new protooncogenes as well as to confirm already known protooncogenes discovered by virtue of their homology to viral oncogenes (7, 8). A tumor suppressor may be scored if a retrovirus lands within a gene and truncates or destroys it. In these cases, the suppressor may be haplo-insufficient, or alternatively, the mutation on the other allele is provided spontaneously by the mouse. The integration event may also lead to more complex consequences, such as a dominant negative effect of the truncated gene product or the transcription of anti-sense or microRNA.

In a screen with T lymphotropic virus SL3-3, a mouse tumor was recovered that contained a proviral integration within intron 1 of the Gpr110 gene (Fig. 1). This integration causes the overexpression of the Gpr110 gene. The human ortholog of this gene is the human GPR110 gene.

### D. Expression of GPR110 and RNA transcript in Human Tumors and in Normal Tissue

The antigenic epitope to the GPR110 antibody is over-expressed in tumors of the human prostate (Fig. 2A), whereas benign prostate hyperplasia (BPH) cells and the normal counterpart of the prostate tumor cells do not or only weakly express the GPR110 protein (Fig. 2B, 2C), demonstrating that an increased distribution and/or localized amount (density) of the protein is diagnostic of human prostate cancer. On occasion, a small subset of BPH cells stain with the GPR110 antibody (Fig. 3A, arrow). These GPR110-positive cells lack PSA expression (Fig. 3B, arrow) and are consistent with being classified as prostate cancer stem cells.

Furthermore, the antigenic epitope to the GPR110 antibody is also over-expressed in human tumors of the lung (Fig. 4A), whereas the normal counterpart of these tumor cells do not or only weakly express the GPR110 protein (Fig. 4B), demonstrating that an increased distribution and/or localized amount (density) of the protein is diagnostic of human lung cancer.

More generally, the invention provides a method for examining tissue or other subject sample, e.g., blood or serum, that normally only weakly expresses or contains GPR110, for the presence and extent of cancer. The method is especially useful for examining prostate and lung tissue, *e.g*., for determining a subtype of prostate and lung cancer in a human patient. In one method directed to examining prostate and lung tissue, the tissue is stained with a labeled antibody specific against a selected domain or epitope of GPR110, *e.g*., fluorescence-labeled antibody (see Section E below), to attach the marker to the tissue cells. Alternatively, the tissue is stained with an unlabeled GPR110 antibody, and the cell-bound antibody complex is labeled with a second labeled antibody, *e.g*., a second antibody carrying a fluorescent, colorimetric or gold-particle reporter. The presence, extent, and stage of prostate or lung cancer in the tissue is then determined based on an increased distribution and/or extent, and typically both, of detectable marker with respect to the distribution and extent of marker in normal prostate or lung cells. Scoring methods for scoring the degree and extent of antibody binding to a histological tissue sample are well known (e.g., "Loda System"). In the present method, intensity scores of 2+ or 3+ and a % cell staining score of 2 or 3 were observed in 35-40% of lung tumors labeled with an anti-GPR110 antibody. For prostate tumors, 20% of tumors had an intensity score of 1 and % cell staining score of 2. For benign prostate hyperplasia samples, 70% had both an intensity score and % cell staining score of 0; 30% had an intensity score of "trace" or 1, with a % cell staining score of 1, when labeled with an anti-GPR110 antibody.

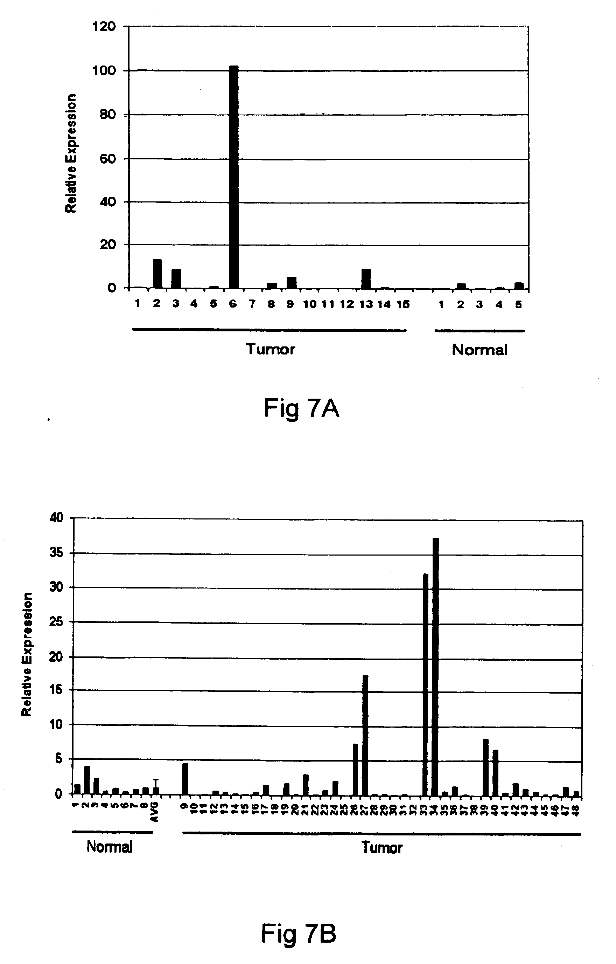

To corroborate GPR110's role in lung cancer, GPR110 RNA transcript levels were measured (using an exon junction (ExJ2-3) Taqman probe) in two different sets of normal and tumor lung tissues. In the first set of tissues, four of the 15 lung adenocarcinoma tumors assayed (2, 3, 6, and 13) showed 8-fold to over 100-fold higher GPR110 RNA levels than normal lung samples (Fig. 7A). In the second set of tissues, six of the 40 lung cancer samples had from 5-fold up to 35-fold overexpression of GPR110 (Fig. 7B) as compared to the normal lung samples. Four of these six elevated samples (27, 33, 34, and 39) were from lung adenocarcinomas while the remaining two (26 and 40) were squamous cell carcinomas. Overall, from both expression experiments, elevated GPR110 expression was seen in ∼20% of lung tumors assayed.

### E. Preparation of Anti-GPR110 Antibody

This section describes production of anti-GPR110 antibodies useful for diagnostic and therapeutic purposes, as described further in the sections below. The anti-GPR110 antibody used in the present invention can be obtained by any variety of conventional methods to produce a monoclonal, polyclonal, and/or recombinant antibody. One preferred antibody, particularly for diagnostic use, is a mouse monoclonal antibody, prepared according to well-known hybridoma methodology. Briefly, human GPR110 may be first obtained, for example, by expressing the GPR110 gene. The purified GPR110 protein acts as an immunogen. Alternatively, a partial peptide of GPR110 can be used as a sensitization antigen. In particular, for generating antibodies specific against a selected epitope or domain of GPR110, a peptide defining that domain or epitope may be used as the immunogen. Exemplary immunogens include the GPR110 epitope represented by amino acid residues within SEQ ID NO:1.

Anti-GPR110 antibodies useful in diagnostic applications may be labeled with a variety of detectable labels, including detectable reporters, such as enzymes for enzyme-linked immunosorbent assays (ELISA), detectable particles, such as gold particles and reporter-carrying liposomes, colorimetric or fluorescent reporters, labels such as quantum dot nanocrystal particles, radiolabels, and labels such as a biotin label by which secondary detectable labels, such as a reporter-labeled streptavidin label can be attached. In some assay formats, an unlabeled anti-GPR110 antibody, for example, a mouse IgG antibody, is detected by reaction with a labeled antibody, *e.g*., a labeled anti-mouse IgG antibody.

For therapeutic uses, human monoclonal antibodies having binding activity to GPR110 can be produced by sensitizing *in vitro* human lymphocytes with GPR110, and causing the sensitized lymphocytes to fuse with the human-derived myeloma cells having a permanent division potential. Alternatively, GPR110 as an antigen can be administered to a transgenic animal having all the repertories of a human antibody gene to obtain anti-GPR110 antibody-producing cells, and then human antibodies for GPR110 may be obtained from the immortalized anti-GPR110 antibody-producing cells.

Also for therapeutic uses, the antibody may be conjugated to (derivatized with) a therapeutic agent, such as a toxin, radiolabeled metal anchored in chelated form, or carrier body, such as liposomes, loaded with an anti-tumor agent, where localization of the antibody carrier on the surface of cells is effective to cause disruption of the cell membrane, *e.g*., by fusion of the carrier with the cell membrane, and release of the therapeutic agent into the cells.

In still other methods, human or humanized antibodies specific against GPR110 antigen can be prepared by recombinant techniques, such as have been reported (see, for example, U.S. Patent Nos. 6,090,382 and 6,258,562).

### F. Diagnostic Methods and Reagents

In one aspect, the invention includes a method of screening for prostate or lung cancer in a human subject, as described in the claims.

If the assayed level is at least threefold greater than normal level, the method may further involve detecting the presence of lung or prostate cancer by means of an independent test for lung or prostate cancer, respectively, in the subject, where the independent test may be carried out prior to, contemporaneous with, or following steps (a) and (b).

For example, where the independent test precedes the GPR110 assay, the independent test may indicate the presence of lung cancer or prostate tumor, and the GPR110 assay is subsequently employed to confirm the presence of the cancer and/or indicate that the cancer is a type characterized by increased level of GPR110 or its transcript. Where the independent test is carried contemporaneously with the GPR110 assay, the method provides an assay result in which two or more cancer markers, including GPR110 or its transcript, are used to detect lung or prostate cancer in a subject. In a third embodiment, the independent test may be carried out subsequent to the GPR110 assay, to verify the diagnosis of lung or prostate cancer, and/or to indicate that the cancer is a type characterized by the presence of elevated level of GPR110 or a variant thereof.

An embodiment of the method in which the subject sample is a blood or serum sample is detailed below. This embodiment involves contacting the sample with an anti-GPR110 antibody specific against a GPR110 epitope, under conditions effective to bind the antibody to the GPR110 epitope, separating antibody bound to the GPR110 epitope from unbound antibody, and detecting the level of the antibody bound to the GPR110 epitope. Solid-strip assay devices having immobilized anti-GPR-Assay for capture of GPR110 in a sample are discussed below. Preferred body-fluid samples are blood, urine, and saliva. Where urine is assayed, the assayed level of GPR110 indicative of prostate or lung cancer is typically in the range of greater than about 1 ng/ml sample fluid.

More generally, detection of GPR110 or its transcript as an aid in diagnosis for the presence, extent, or staging of prostate or lung cancer can be used alone or in combination with the detection and screening of additional marker proteins associated with prostate or lung cancer. Biomarkers or marker proteins refer to any detectable biological molecule in which its altered expression, distribution, or a particular form of the biomarker correlates with the presence, extent, or stage of a physiologic condition, such as a disease state. As understood by those skilled in the art, there need not be a strict association between the biomarker and the physiologic condition, but only that a statistically significant association is present between the biomarker and the physiologic condition. Additional biomarkers can be selected from, among others, prostate specific antigen (PSA), including total or free PSA or both, glypican 3 protein (GPC3) and combinations thereof.

Where the additional biomarker is PSA, the levels or distribution of PSA can be determined, as noted above, for total PSA, free PSA, or combination thereof, according to methods in the art. In some cases, the levels of total PSA and fPSA, generally expressed in the art as the ratio of fPSA to total PSA, can be used in combination with detection of GPR110. Testing for PSA can be on the same or different biological specimen used to detect the GPR110. For example, for use in screening male human subjects for prostate cancer, step (a) in the method described above may include reacting the sample with an antibody specific against prostate-specific antigen (PSA), to produce a reaction product related to the level of PSA in the sample, and step (b) may include determining, level of PSA when compared with a normal range of PSA in non-cancerous human samples.

The additional biomarker GPC3 is characterized as a heparin sulfate proteoglycan anchored to the cell membrane via glycosylphosphatidylinositol. The protein has a molecular weight of 65.6 kDa and the polypeptide chain has 580 amino acid residues. The heparin sulfate chain of the proteoglycans interacts with heparin-binding growth factors and thus serves as a co-receptor in cell signaling, although GPC3 might bind also in a different way. In embryonic development, GPC3 modulates BMP and EGF-mediated effects during renal branching morphogenesis. It also controls cellular responses to BMP4 in limb patterning and skeletal development. The levels of GPC3 protein are increased in prostate cancer tissues. Its use as a specific biomarker for prostate and methods of its detection, such as by antibodies specifically binding to GPC3, are described in co-owned US application Serial No. 11/325,847. A method for detecting GPC3 can employ antibodies that specifically bind to GPC3, such as polyclonal, monoclonal, or recombinant antibodies. An exemplary antibody, particularly for diagnostic use, includes a mouse monoclonal antibody, prepared according to well-known hybridoma methodology. Briefly, human GPC3 may be first obtained, for example, by expressing the GPC3 (MXR7) gene as disclosed by Lage, H. et al (Gene 188 (1997), 151-156). The purified GPC3 protein is used as an immunogen. Alternatively, a partial peptide of GPC3 can be used as a sensitization antigen. The partial peptide can be obtained by chemical synthesis from the amino acid sequence of human GPC3. By way of example and not limitation, exemplary GPC3 sequences that may be employed include, among others, DLFIDKKVLKVAHVEHEET, SEQ ID NO:3 (amino acid residues 365 to 383, encoded by exon 4) and LAYDLDVDDAPGNSQQ, SEQ ID NO:4 (amino acid residues 526 to 541, encoded by exon 8). Other peptides for producing antibodies directed against GPC3 will be apparent to the skilled artisan in view of the known sequence of GPC3.

The assay may be carried out by any of a variety of assay methods used for detecting body-fluid antigens, including ELISA techniques, homogeneous assays, for example, involving fluorescence quenching, and a variety of solid-phase sandwich assays in which the GPR110 antigen is captured by an anti-GPR110 antibody carried on a solid support, and the immobilized antigen-antibody complex is labeled with a second anti-GPR110 antibody, *e.g*., a second antibody carrying a colorimetric or gold-particle reporter.

Figs. 5A and 5B illustrate a solid-phase assay strip that may be used in the method of the invention and is suitable for carrying out a sandwich immunoassay of the type just mentioned, and shown in initial and final assay states, respectively. The strip, indicated generally at **10,** includes a porous support or pad **12** having a sample-application zone **14** in an upstream region of the support and a sample-detection zone **16** in a downstream region. The sample-application zone includes a detectable anti-GPR110 antibody reagent, *e.g*., anti-GPR110 antibodies labeled with gold particles, and carried in the zone in an unbound, *i.e*., non-immobilized form. This reagent is indicated by solid circles, such as at **18.** Anti-GPR110 antibodies, which may be the same or different from those in the labeled antibody reagent, are immobilized to the solid support within the detection zone, and are indicated by the "Y" shapes, such as at **20.**

Also shown is a reference zone **22** which is located adjacent the detection zone and has one or more colored or shaded regions corresponding to different assay levels of GPR110 in a body-fluid sample. In the embodiment shown, zone **22** includes three regions **22a, 22b,** and **22c,** corresponding to an assayed level of GPR110 (a) below that associated with cancer, (b) corresponding to a lower threshold level associated with cancer, and (c) a level that is substantially higher, *e.g*., 2-3 times, higher than the threshold layer in region **22b,** respectively. These three regions provide a known standard indicator against which the level of detectable reaction produced can be assessed as a level associated with prostate or lung cancer. Together, the assay strip and reference zone constitute an assay device for use in screening for prostate or lung cancer in a human subject, or for staging treatment of prostate or lung cancer in a human subject.

In operation, a known volume of a body-fluid sample to be tested is added to the sample-application zone of the strip, where it diffuses into the zone, allowing the antibody reagent to react with GPR110 antigen in the sample to form an antigen-antibody complex. This complex and unbound antibody reagent then migrate downstream by capillarity toward the detection zone, where the antigen-antibody complex is captured by the immobilize antibody and the unbound reagent is carried to the end of the support, as indicated at **24.** As can be appreciated, the higher the concentration of antigen in the body fluid, the higher the density of captured reagent in the detection zone and the greater the color or intensity in this zone. This color or intensity produced in the detection zone is compared with the standards in the reference zone to determine a qualitative level of GPR110 associated with the presence or absence of prostate or lung cancer. If an above-threshold level of GPR110 is observed in the assay, the subject can be classified in a higher-probability category for the presence of cancer and the subject may be recommended for additional testing and/or more frequent testing.

The assay device that may be used in a method of the invention includes an assay strip like that, described above, but where the known-reference indicator is provided by a strip-reader instrument reader having (i) a reader slot for receiving the assay strip, (ii) a light source and an optical detection, *e.g*., a spectrophotometric detector, for detecting an assay-related optical condition at the detection zone of the assay strip, (iii) an electronics or processor unit which records and processes a signal from the optical detector, and converts the signal to an assayed level of GPR110, and (iv) a user display screen or window. The instrument may report the actual GPR110 body-fluid sample detected, allowing the operator to compare the displayed value with known standard indicator levels provided with the assay strip or instrument, to assess whether the subject has an increased GPR110 level associated with prostate or lung cancer, or to assess the possible stage of the cancer, for purposes of treatment design. Alternatively, the instrument itself may contain stored known-standard indicator levels which can be compared internally with an assayed level to generate an output that indicates whether an increased GPR110 level associated with prostate or lung cancer has been detected, or to indicate the stage of the cancer.

It will be appreciated how the just-described assay device may be modified for detecting multiple marker proteins, and in particular, for screening of or detection for prostate cancer, the GPR110 protein in combination with total PSA, free PSA and/or GPC3. Each marker may be measured in a separate strip to which the marker-specific antibody is confined, and the device may further include multiple reference zones, each providing a known-standard indicator against which the level of detectable marker protein reaction produced can be assessed, in combination with the level of detectable GPR110, as an indicator of prostate cancer.

Alternative, in an electronic assay device, the reference values of the multiple markers may be stored or represented as tuples of values, e.g., pairs of values, where each value in a tuple represents an Indicator value for cancer for a given marker, so that by analyzing the multi-values assay results against the stored tuple values, the device can make a determination of cancer risk based on correlations with a greater number of markers.

### Sequence Listing

SEQ ID NO: 1 N-terminal extracellular domain of human GPR110 protein (isoform 1) (residues 1-590)
SEQ ID NO: 2 human GPR110 protein (isoform 1) (residues 1-910)
SEQ ID NO: 3 (amino acid residues 365 to 383, encoded by exon 4)
   DLFIDKKVLKVAHVEHEET
SEQ ID NO: 4 (amino acid residues 526 to 541, encoded by exon 8)
   LAYDLDVDDAPGNSQQ

## Claims

1. A method of screening for lung or prostate cancer in a human subject, comprising
(a) assaying the level of human GPR110 in a subject blood or serum sample, by contacting said sample with an anti-GPR110 antibody specific against a GPR110 epitope represented by amino acid residues within SEQ ID NO:1, under conditions effective to bind the antibody to the GPR110 epitope, separating antibody bound to the GPR110 epitope from unbound antibody, and detecting the level of the antibody bound to the GPR110 epitope
(b) determining if the detected level of antibody bound to the GPR110 epitope is at least threefold greater than that of anti-GPR110 antibody bound to the GPR110 epitope present in blood or serum samples obtained from normal individuals.

2. The method of Claim 1, wherein step (a) includes applying the blood or serum sample body fluid to a solid-phase immunoassay device, where the level of GPR110 in the sample is indicated qualitatively by a colorimetric or fluorometric indicator, and the determining step includes comparing the indicator with a known standard.

3. The method of Claim 1, further comprising detecting a depressed or elevated level of a biological marker or indicator that is diagnostic of lung or prostate cancer.

4. The method of Claim 3 for screening for prostate cancer in a human male subject, further comprising reacting a subject body-fluid sample with an antibody specific against at least one biological marker or indicator diagnostic of prostate cancer selected from one of total prostate specific antigen (PSA), free PSA, and glypican 3 protein (GPC3), and determining, as an indicator of prostate cancer, whether the subject has an increased level of at least one of said marker protein.

## Patentansprüche

1. Verfahren zum Screening auf Lungen- oder Prostatakrebs bei einem menschlichen Subjekt, das Folgendes umfasst:
(a) Untersuchen der Höhe von menschlichem GPR110 in einer Blut- oder Serumprobe des Subjekts durch Inkontaktbringen der genannten Probe mit einem anti-GPR110-Antikörper, der spezifisch gegen ein GPR110-Epitop ist, das durch Aminosäurereste innerhalb von SEQ-ID-NR. 1 dargestellt ist, unter Bedingungen, die zur Bindung des Antikörpers zum GPR110-Epitop wirksam sind, Abtrennen des zum GPR110-Epitop gebundenen Antikörpers vom ungebundenen Antikörper und Nachweisen der Höhe des zum GPR110-Epitop gebundenen Antikörpers;
(b) Bestimmen, ob die nachgewiesene Höhe an zum GPR110-Epitop gebundenen Antikörper mindestens dreimal größer ist als die des zum GPR110-Epitop gebundenen anti-GPR110-Antikörpers ist, der in Blut- oder Serumproben vorliegt, die von normalen Individuen erhalten werden.

2. Verfahren nach Anspruch 1, worin der Schritt (a) das Verwenden der Körperflüssigkeit der Blut- oder Serumprobe in einem Gerät für Festphasen-Immunoassay einschließt, worin die Höhe an GPR110 in der Probe qualitativ durch einen kolorimetrischen oder fluorometrischen Indikator angezeigt wird und der Bestimmungsschritt das Vergleichen des Indikators mit einem bekannten Standard einschließt.

3. Verfahren nach Anspruch 1, das weiter das Nachweisen einer gesenkten oder erhöhten Höhe eines biologischen Markers oder Indikators, der zur Diagnose von Lungen- oder Prostatakrebs dient, umfasst.

4. Verfahren nach Anspruch 3 zum Screening auf Prostatakrebs bei einem menschlichen männlichen Subjekt, das weiter Folgendes umfasst: Reagieren einer Körperflüssigkeitsprobe des Subjekts mit einem Antikörper, der mindestens gegen einen biologischen, zur Diagnose von Prostatakrebs dienenden Marker oder Indikator spezifisch ist, der aus einem des gesamten Prostata-spezifischen Antigens (PSA), freien PSA und Glypican-3-Proteins (GPC3) ausgewählt ist, und Bestimmen, als Indikator von Prostatakrebs, ob das Subjekt eine angestiegene Höhe von mindestens einem der genannten Markerproteine aufweist.

## Revendications

1. Méthode de criblage d'un cancer du poumon ou de la prostate chez un sujet humain, comprenant les étapes consistant à :
(a) doser le taux de GPR110 humain dans un échantillon de sang ou de sérum du sujet, par la mise en contact dudit échantillon avec un anticorps anti-GPR110 spécifique d'un épitope de GPR110 représenté par les résidus d'acides aminés au sein de SEQ ID n° 1, dans des conditions efficaces pour lier l'anticorps à l'épitope de GPR110, la séparation de l'anticorps lié à l'épitope de GPR110 de l'anticorps non lié, et la détection du taux d'anticorps lié à l'épitope de GPR110 ;
(b) déterminer si le taux détecté d'anticorps lié à l'épitope de GPR110 est au moins trois fois plus important que celui de l'anticorps anti-GPR110 lié à l'épitope de GPR110 présent dans des échantillons de sang ou de sérum obtenus auprès d'individus normaux.

2. Méthode selon la revendication 1, dans laquelle l'étape (a) comporte l'application de l'échantillon de fluide corporel sanguin ou sérique à un dispositif d'immunoessai en phase solide, où le taux de GPR110 dans l'échantillon est indiqué de manière qualitative par un indicateur colorimétrique ou fluorimétrique, et l'étape de détermination comporte la comparaison de l'indicateur avec un étalon connu.

3. Méthode selon la revendication 1, comprenant en outre la détection d'un taux en baisse ou élevé d'un indicateur ou marqueur biologique qui constitue un diagnostic d'un cancer du poumon ou de la prostate.

4. Méthode selon la revendication 3, destinée au criblage d'un cancer de la prostate chez un sujet mâle humain, comprenant en outre les étapes consistant à faire réagir un échantillon d'un fluide corporel d'un sujet avec un anticorps spécifique d'au moins un indicateur ou marqueur biologique de diagnostic du cancer de la prostate choisi parmi l'un parmi l'antigène spécifique de la prostate total (PSA), le PSA libre, et la protéine glypican-3 (GPC3), et déterminer, comme indicateur du cancer de la prostate, si le sujet possède un taux accru d'au moins l'une desdites protéines marqueur.
